# EUROPEAN PATENT APPLICATION

(11) **EP 4 147 716 A1**
(43) Date of publication of application: **15.03.2023**
(21) Application number: 21195979.6
(22) Date of filing: 10.09.2021
(51) Int. Cl.: A61K 39/12, C07K 16/10

(54) **ANTI-VIRUS MOIETY IMMOBILISED IN A MATRIX**

(71) Applicant: Samatva Research Corporation, Thunder Bay, Ontario P7E 6E7 (CA)
(72) Inventor: The designation of the inventor has not yet been filed
(74) Representative: J A Kemp LLP

(57) **Abstract**

The invention relates to hygiene products for filtering or removal of virus particles and/or fragments thereof using an anti-virus moiety immobilised in a matrix.

## Description

### Field of the Invention

The invention relates to products for the removal of virus particles and/or fragments thereof, and methods and uses thereof.

### Background to the Invention

To survive within a population, viruses must spread from an infected host to a susceptible host in a process known as viral transmission. Although they cannot replicate independently outside a host, virus particles can remain infectious outside the body and persist in the air and on surfaces for up to several hours. Transmission can be facilitated by, for example, respiratory droplets, airborne particles, and contaminated surfaces known as fomites.

Respiratory droplets are expelled when an infected person coughs or sneezes, for example. Respiratory droplets are approximately 5-10µm in diameter and transmission can occur when a person is in close contact with an infected host.

Small droplets of less than 5µm in diameter (often known as droplet nuclei or aerosols) are responsible for airborne transmission. These airborne particles remain suspended in air for significant periods of time and can readily be inhaled.

Additionally, respiratory droplets and airborne particles expelled by infected individuals can contaminate surfaces and objects, creating fomites. Viral transmission may therefore occur indirectly through touching such fomites, followed by touching the mouth, nose or eyes.

Strategies to reduce the transmission of viruses include the use of filters, masks and surface cleaners. HEPA filters and N95 masks are designed to provide protection against both respiratory droplets and airborne particles. They block approximately 99.9% of all particles that are at least 0.3 µm in diameter, and they are thought to be capable of blocking approximately 99.8% of particles that are at least 0.1 µm in diameter. N95 masks can also reduce the inhalation of airborne virus particles and droplets into the user's airway. Additionally, surface cleaners and hand sanitisers are effective at dissolving the viral lipid coat, thus preventing transmission by eliminating viruses from fomites.

The importance of virus transmission preventative strategies is highlighted in the coronavirus disease 2019 (COVID-19) pandemic, which is a severe acute respiratory syndrome caused by SARS-CoV-2 and has posed unprecedented challenges to public health on a global scale. With the emergence of variant SARS-CoV-2 strains which can spread even more widely and quickly than the original SARS-CoV-2 strain, there is an urgent need to identify effective preventative measures to reduce virus transmission.

As demonstrated during the COVID-19 pandemic, filters, masks, surface cleaners and hand sanitisers each have limitations. For example, HEPA filters and N95 masks are expensive and need to be replaced frequently. N95 masks containing exhalation valves allow exhaled air to be propelled outwards, meaning that particles in exhaled air travel further than if a regular (paper or cotton) mask were used. Additionally, mechanical action caused by speaking, shouting, sneezing or coughing can cause significant emission of virus particles from the fibres of regular masks. Regular masks and N95 masks are also often worn incorrectly, and can give users a false sense of security. Surface cleaners and alcohol-based hand sanitisers contain chemicals that are toxic when misused, with the latter leading to potential long-term health effects, such as increased chance of antimicrobial resistance.

It is therefore an object of the invention to identify further and improved methods and products for removing or filtering virus particles and/or fragments thereof, e.g. from the air or from fomites.

It is also an object of the invention to identify further and improved methods and products for preventing the transmission of viruses, such as SARS-CoV-2.

### Summary of the Invention

Anti-virus moieties immobilised in hygiene products can be used to specifically "trap" target virus particles and/or fragments thereof. Hence, such immobilised anti-virus moieties are particularly useful for preventing virus transmission, e.g. by filtering or removing virus particles and/or fragments thereof from, e.g. the environment or a surface. The hygiene products and components thereof can be easily prepared in an economic fashion and are non-toxic.

As shown in the Examples, anti-virus moieties, when immobilised in a matrix, are effective in binding to virus pseudoparticles with high affinity and for an extended period of time. In particular, an anti-virus moiety was prepared in the form of a single-chain variable fragment (scFv) derived from human monoclonal antibody (mAb) which recognises the receptor binding domain (RBD) of the spike protein in SARS-CoV-2. The anti-virus moiety was coupled to a cellulose-binding domain. The fusion proteins used in the Examples are referred to herein as Karunyam proteins.

Each of the Karunyam proteins used in the Examples was found to specifically bind to the RBD of the spike protein. The binding affinity between each of the Karunyam proteins and the RBD was found to be similar to the binding affinity between the ACE2 receptor and the RBD, and the binding affinity between the monoclonal antibodies from which the scFvs were derived and the RBD.

Furthermore, when the Karunyam proteins were immobilised on cellulose beads, they were effective in binding to virus pseudoparticles (RBD-spike protein conjugated to FITC nanoparticles) quickly and the binding remains intact even after one week. The Karunyam proteins immobilised on cellulose beads were also capable of retaining the virus pseudoparticles after centrifugation at high speed.

These findings therefore confirm the effectiveness of anti-virus moieties, when immobilised on a matrix, in binding to virus particles, and hence their usefulness as hygiene products for preventing virus transmission, e.g. by filtering and removing viruses from the environment or a surface.

Accordingly, the invention provides an anti-virus moiety immobilised in a matrix, wherein the matrix is suitable for use as a hygiene product.

The invention also provides a fusion protein comprising: (i) an anti-virus moiety, such as an anti-virus moiety described herein, and (ii) a matrix-binding domain; optionally wherein the fusion protein further comprises a linker between the anti-virus moiety and the matrix-binding domain.

The invention also provides a matrix comprising the anti-virus moiety or the fusion protein of the invention immobilised therein, optionally wherein the matrix is cellulose, starch, collagen, insect shells, mucous membranes, or an inorganic matrix.

The invention also provides a hygiene product comprising the anti-virus moiety, the fusion protein or the matrix of the invention.

The invention also provides a method of filtering or removing virus particles and/or fragments thereof from the environment or a surface, comprising applying the immobilised anti-virus moiety, the fusion protein, the matrix, or the hygiene product of the invention to filter or remove virus particles and/or fragments thereof from the environment or a surface.

The invention also provides the use of the immobilised anti-virus moiety, the fusion protein, the matrix, or the hygiene product of the invention to filter or remove virus particles and/or fragments thereof from the environment or a surface.

The invention also provides a method of preventing virus transmission comprising filtering or removing virus particles and/or fragments thereof from the environment or a surface according to the method or use of the invention.

### Brief Description of the Figures

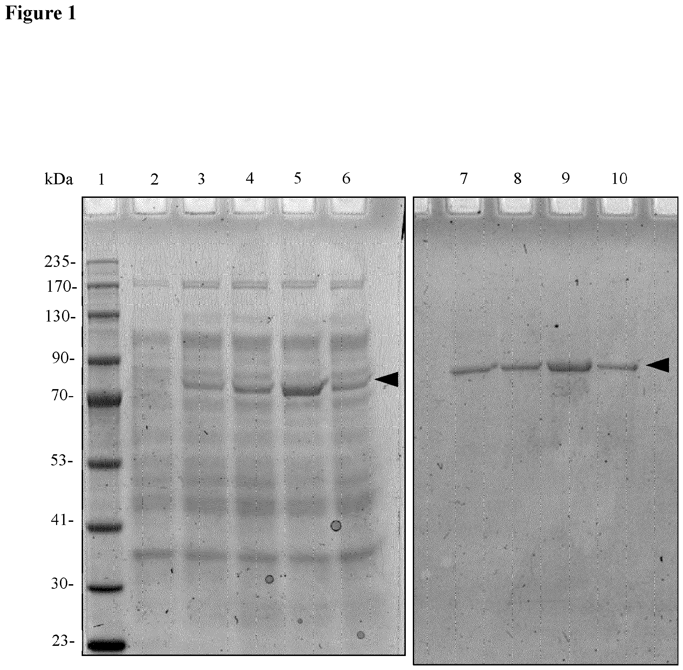

**Figure 1** shows a 4-12% SDS-PAGE of proteins expressed from bacteria containing a vector encoding Karunyam 9. Lane 1 shows molecular weight markers. Lane 2 is whole cell lysate prior to induction of vector expression. Lanes 3-6 are whole cell lysates of four preparations following induction of vector expression. Lanes 7-10 are four separate preparations of Karunyam 9 following purification. Arrows indicate the Karunyam 9 protein.

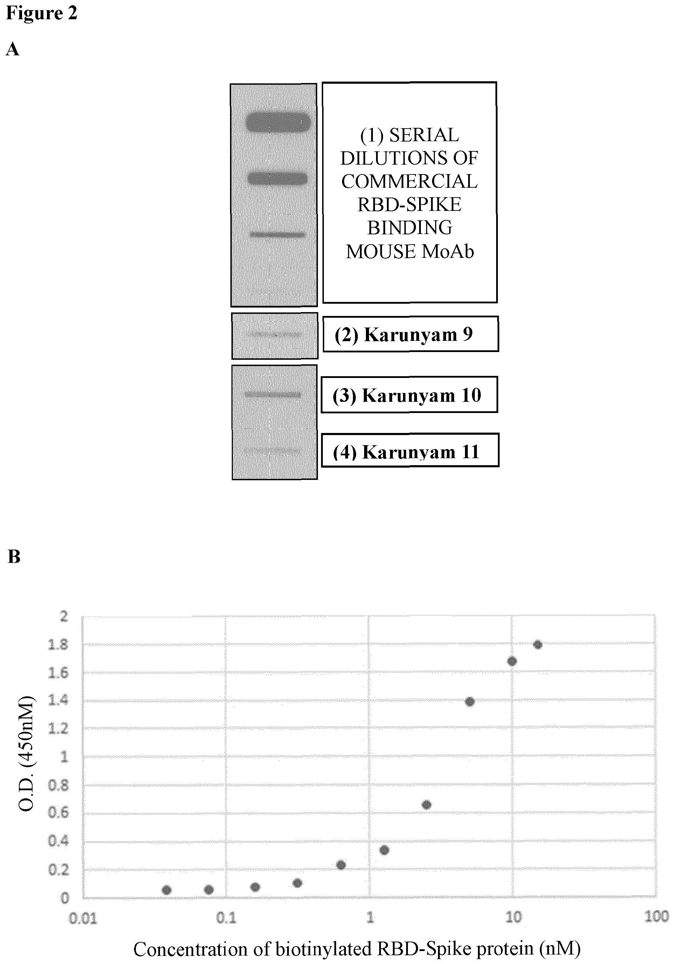

**Figure 2** shows that anti-virus Karunyam proteins can bind to the RBD-Spike protein with high specificity and high affinity. **A** is a far-western blot of: (1) serial dilutions of commercial RBD-Spike binding mouse monoclonal antibodies; and (2)-(4) Karunyam proteins probed with RBD-Spike protein conjugated to HRP. **B** is a graph depicting the results of an ELISA with immobilised Karunyam 9 probed with RBD-Spike protein conjugated to HRP

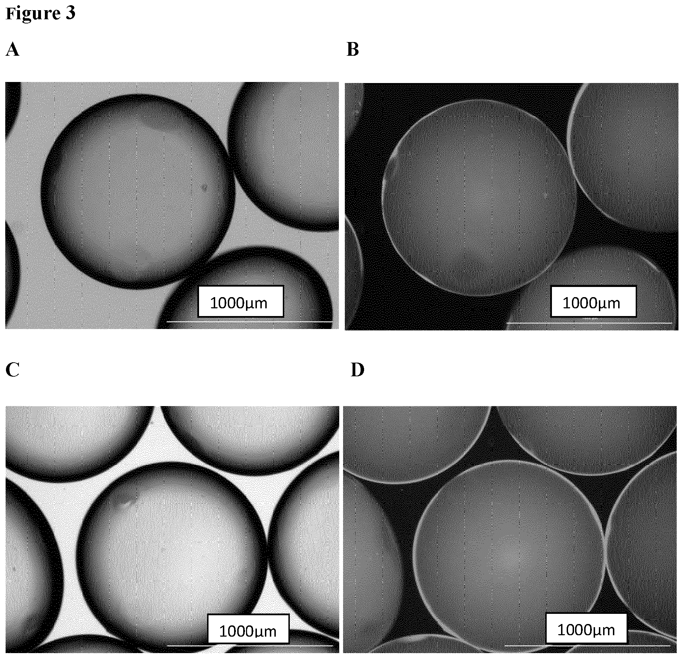

**Figure 3** shows that Karunyam 9 immobilised on cellulose beads bind to RBD-Spike protein conjugated to FITC nanoparticles (virus pseudoparticles) for extended periods of time. **A** and **B** shows the cellulose beads under light and fluorescence microscopy respectively, after 5 minutes. **C** and **D** shows the cellulose beads under light and fluorescence microscopy respectively, after one week in solution.

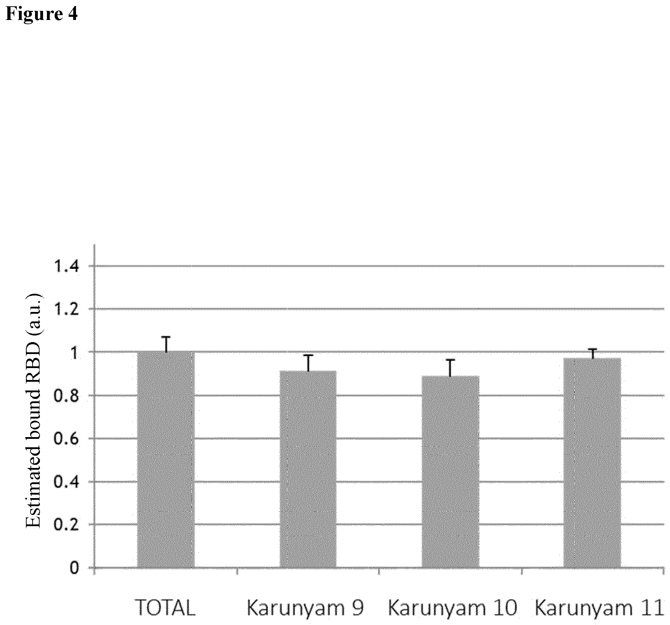

**Figure 4** shows that Karunyam proteins immobilised on cellulose beads retain virus pseudoparticles with high affinity. The amount of virus pseudoparticles bound to each immobilised Karunyam protein was estimated by subtracting the unbound fraction from the total virus pseudoparticle as estimated by immunochromatography strip analysis. The unbound fraction was estimated using a competitive ELISA.

### Detailed Description of the Invention

### Anti-virus moiety

An anti-virus moiety useful with the invention is capable of specifically binding to a target virus particle and/or a fragment thereof. Typically, the anti-virus moiety specifically binds to the target virus particle and/or a fragment thereof with high affinity.

By referring to "specifically binding", it is meant that the anti-virus moiety recognises and binds the target virus and/or a fragment thereof, but does not substantially recognise and bind other molecules, e.g. in a sample such as a biological sample or an environment sample.

The anti-virus moiety may be a protein or a mixture of proteins, but may also be an aptamer, affimer, molecularly imprinted polymer (MIP), or a nucleic acid.

The anti-virus moiety is typically an antibody. The term "antibody" referred to herein may comprise a complete antibody having full length heavy and light chains, or an antigen-binding fragment thereof. For example, the anti-virus moiety may be a full-length antibody.

The anti-virus moiety useful with the invention may be an antigen-binding fragment. An antigen-binding fragment of the invention binds to the same epitope of the parent antibody, *i.e.* the antibody from which the antigen-binding fragment is derived. An antigen-binding fragment of the invention typically retains the parts of the parent antibody that interact with the epitope. The antigen-binding fragment typically comprises the complementarity-determining regions (CDRs) that interact with the antigen, such as one, two, three, four, five or six CDRs. In some embodiments, the antigen-binding fragment further comprises the structural scaffold surrounding the CDRs of the parent antibody, such as the framework regions (e.g. FR1, 2, or 3) and/or the variable region domains of the heavy and/or light chains. Typically, the antigen-binding fragment retains the same or similar binding affinity to the antigen as the parent antibody.

For example, an antigen-binding fragment may be a Fab, modified Fab, Fab', modified Fab', F(ab')₂, Fv, single domain antibody (*e.g*. VH or VL or VHH), single chain variable fragment (scFv), bi, tri or tetra-valent antibody, Bis-scFv, diabody, triabody, tetrabody or epitope-binding fragments of any of the above (see for example References 1 and 2). The methods for creating and manufacturing antibody fragments are well known in the art (see for example Reference 3).

The inventors found that scFvs are particularly useful with the invention, as shown in the Examples. Hence, the anti-virus moiety useful with the invention may be a scFv.

The anti-virus moiety may bind to any epitope in a target virus particle. For example, it may bind to a neutralising epitope of the target virus particle, *e.g.* it may neutralise the activity of the virus. Alternatively, the anti-virus moiety may bind to a nonneutralising epitope.

The target virus particle may be coronavirus, Ebola virus, respiratory syncytial virus (RSV), influenza virus, adenovirus, human rhinovirus, human metapneumovirus, parainfluenza virus, hantavirus, Bocavirus, or Marburg virus. For example, the target virus particle may be coronavirus, e.g. SARS-CoV-2, SARS-CoV-1 or Middle Eastern Respiratory Syndrome (MERS).

The target virus particle may be SARS-CoV-2. SARS-CoV-2 is a beta coronavirus, which is related to SARS-CoV and MERS coronaviruses. Embedded in the membrane envelope of both SARS-CoV-2 and SARS-CoV-1 viruses are multiple copies of a glycoprotein known as the spike protein, which recognises the angiotensin converting enzyme 2 (ACE2) receptor on host cells to facilitate cell entry. The specific region of the spike protein that interacts with the ACE2 receptor, and therefore the region responsible for attaching the virus to host cells, is known as the receptor binding domain (RBD).

The epitope in the target virus particle may be in the spike protein of a coronavirus, e.g. SARS-CoV-2.

The epitope may be in the the S1 subunit of the spike protein, such as the receptor binding domain (RBD) or N-terminal domain (NTD). For example, the epitope may be in the RBD of the spike protein.

The anti-virus moiety may be a scFv that binds to the RBD in the spike protein of SAR-CoV-2.

Known antibodies in the art that specifically bind to coronavirus, e.g. SARS-CoV-2, are useful with the invention. For example, antibodies P2B-2F6, P2C-1F11, P2C-1A3, P2C-1D5, P2A-1A10, P2A-1B3, P2C-1C8, P2A-1A8, P2C-1E1, P2A-1A9, P2C-1C10, P2B-2G4, P2B-2G11, P1A-1C10, P1A-1C7, P1A-1D1, P1A-1B2, P1A-1C1, m396, 80R and/or CR3022, as disclosed in References 4 and 5, are useful with the invention. For example, the anti-virus moiety useful with the invention may be any of these antibodies, a fragment thereof, and/or a molecule derived therefrom.

For example, the anti-virus moiety may be derived from P2B-2F6, P2C-1F11 or P2C-1A3, such as the scFvs in the Examples.

Hence, an anti-virus moiety useful with the invention (e.g. an antibody) may comprise the complementary determining regions: CDRH1, CDRH2, CDRH3, CDRL1, CDRL2 and CDRL3 as set out in SEQ ID NOs: 11, 13, 15, 17, 19 and 21 respectively. The anti-virus moiety may additionally comprise the framework regions: FRH1, FRH2, FRH3, FRL1, FRL2 and FRL3 as set out in SEQ ID NOs: 10, 12, 14, 16, 18 and 20, respectively. The anti-virus moiety (e.g. an antibody) may comprise or consist of an amino acid sequence having ≥70% (i.e. 70% or more), ≥80%, ≥90%, ≥95%, ≥96%, ≥97%, ≥98%, ≥99% or 100% sequence identity with SEQ ID NO: 1, provided that it is capable of specifically binding to the target virus particle and/or fragment thereof. The anti-virus moiety may be a scFv consisting of SEQ ID NO: 1, i.e. as referred to herein as scFv 9.

An anti-virus moiety useful with the invention (e.g. an antibody) may comprise CDRH1, CDRH2, CDRH3, CDRL1, CDRL2 and CDRL3 as set out in SEQ ID NOs: 23, 25, 27, 29, 31 and 33 respectively. The anti-virus moiety may additionally comprise a FRH1, FRH2, FRH3, FRL1, FRL2 and FRL3 as set out in SEQ ID NOs: 22, 24, 26, 28, 30 and 32, respectively. The anti-virus moiety (e.g. an antibody) may comprise or consist of an amino acid sequence having ≥70% (i.e. 70% or more), ≥80%, ≥90%, ≥95%, ≥96%, ≥97%, ≥98%, ≥99% or 100% sequence identity with SEQ ID NO: 2, provided that it is capable of specifically binding to the target virus particle and/or fragment thereof. The anti-virus moiety may be a scFv consisting of SEQ ID NO: 2, i.e. as referred to herein as scFv 10.

An anti-virus moiety useful with the invention (e.g. an antibody) may comprise CDRH1, CDRH2, CDRH3, CDRL1, CDRL2 and CDRL3 as set out in SEQ ID NOs: 35, 37, 39, 41, 43 and 45 respectively. The anti-virus moiety may additionally comprise a FRH1, FRH2, FRH3, FRL1, FRL2 and FRL3 as set out in SEQ ID NOs: 34, 36, 38, 40, 42 and 44, respectively. The anti-virus moiety may comprise or consist of an amino acid sequence having ≥70% (i.e. 70% or more), ≥80%, ≥90%, ≥95%, ≥96%, ≥97%, ≥98%, ≥99% or 100% sequence identity with SEQ ID NO: 3, provided that it is capable of specifically binding to the target virus particle and/or fragment thereof. The anti-virus moiety may be a scFv consisting of SEQ ID NO: 3, i.e. as referred to herein as scFv 11.

Typically, the anti-virus moiety binds to the epitope, *e.g.* in the RBD of SARS-CoV-2, with high affinity, e.g. with a K_{D} value of ≤100nM, ≤50nM, ≤10nM, ≤5nM, ≤1nM, ≤0.5nM, or ≤0.1nM. For example, the K_{D} value may be between 1 to 10 nM, e.g. between 4nM to 8 nM. For example, the K_{D} values of some of the anti-virus moieties of the invention are provided in Tables 3 and 4. Binding affinity (K_{D}) can be analysed by any suitable means known in the art, for example, by ELISA or Surface Plasmon Resonance, *i.e.*, detection of real-time biospecific interactions by detection of changes in protein concentration in a biosensor matrix using, for example, the BIACORE system (Pharmacia Biosensor AB, Uppsala, Sweden).

The anti-virus moiety is typically prepared in an expression system. Expression systems useful for protein production are known in the art, e.g. mammalian (e.g. CHO cells, 293 cells), insect, yeast, bacterial (e.g. *E. coli*), algal, and cell-free protein expression systems, and any of these expression systems can be used to express an anti-virus moiety useful with the invention.

The anti-virus moiety is typically soluble in the expression system, e.g. *E. coli.* For example, the anti-virus moiety may be naturally soluble in the physiological condition of the expression system, e.g. in *E. coli* cells. The anti-virus moiety may be fused to a solubility tag to increase its solubility in the physiological condition of the expression system. Useful solubility tags are known in the art, e.g. Glutathione-S-transferase (GST), Maltose Binding Protein (MBP) and Thioredoxin (TRx), and any of these solubility tags can be used to express an anti-virus moiety useful with the invention. The solubility tag may be fused to the N- or C-terminal of anti-virus moiety, for example.

For example, the anti-virus moiety may be fused to GST comprising or consisting of an amino acid sequence having ≥70% (i.e. 70% or more), ≥80%, ≥90%, ≥95%, ≥96%, ≥97%, ≥98%, ≥99% or 100% sequence identity with SEQ ID NO: 5.

It is within the teaching herein that the anti-virus moiety may be fused to an affinity tag for protein purification e.g. HA-tag which correspond to amino acids 98 to 106 of human influenza hemagglutinin, polyhistidine (His) (SEQ ID NO: 4), c-myc and FLAG. The affinity tag may be fused to the N- or C-terminal of anti-virus moiety, for example.

Hence, the invention also provides a fusion protein comprising: (i) an anti-virus moiety described herein and (ii) a solubility tag and/or an affinity tag. The solubility tag may be GST comprising or consisting of an amino acid sequence having ≥70% (i.e. 70% or more), ≥80%, ≥90%, ≥95%, ≥96%, ≥97%, ≥98%,≥99% or 100% sequence identity with SEQ ID NO: 5. The affinity tag may be polyhistidine comprising or consisting of SEQ ID NO: 4. The solubility tag and/or the affinity tag may be fused to the N- or C-terminal of anti-virus moiety. For example, the fusion protein may comprise the solubility tag and the affinity tag fused to the N-terminal of anti-virus moiety.

The anti-virus moiety is typically functional in the expression system, e.g. *E. coli.* For example, the anti-virus moiety is capable of specifically binding to its target, e.g. with high affinity as described herein. The anti-virus moiety expressed from the expression system may be modified and/or manipulated (e.g. subjected to a denaturation and renaturation protocol) to obtain a functional anti-virus moiety.

Hence, the invention also provides a method of preparing an anti-virus moiety, comprising fusing the anti-virus moiety with a solubility tag and optionally subjecting the anti-virus moiety to a denaturation and renaturation protocol.

Methods of testing the functional properties of an anti-virus moiety include determining its binding affinity to the target virus particle. Techniques for determining binding affinity is known in the art, e.g. by ELISA or Surface Plasmon Resonance, *i.e.,* detection of real-time biospecific interactions by detection of changes in protein concentration in a biosensor matrix using, for example, the BIACORE system (Pharmacia Biosensor AB, Uppsala, Sweden).

### The matrix and the matrix-binding domain

A matrix of the invention is capable of retaining target virus particles and/or fragments thereof via an anti-virus moiety as described herein. The anti-virus moiety useful with the invention is immobilised in a matrix.

The matrix may be solid or liquid in nature.

The matrix may be cellulose, starch, collagen, insect shells, mucous membrane, or inorganic matrix.

The cellulose matrix may be a cellulose bead, a paper product, cotton fabric or a cellulose solution.

The anti-virus moiety may be immobilised in a matrix in an irreversible manner. Alternatively, the anti-virus moiety may be immobilised in a matrix in a reversible manner.

The anti-virus moiety may be immobilised by methods well-known in the art, e.g. by cross-linking, covalent binding or physically adsorbing the anti-virus moiety to the matrix.

The anti-virus moiety may be immobilised in the matrix via a matrix-binding domain. For example, the anti-virus moiety may be coupled, e.g. chemically coupled or fused, to a matrix-binding domain without altering the binding capacity of the anti-virus moiety. Hence, the invention provides an anti-virus moiety described herein in the form of a fusion protein comprising an anti-virus moiety and a matrix-binding domain, as explained further below.

The matrix-binding domain is typically a peptide or a mixture of peptides that is capable of specifically binding to a particular matrix.

The matrix-binding domain may bind the matrix with high affinity, e.g. with a K_{D} value of less than 1µM, e.g. between 10⁻⁶ M to 10⁻¹⁶ M, such as between 10⁻⁶ M to 10⁻¹⁵ M. For example, the K_{D} value is between 0.1 µM to 1 µM.

The anti-virus moiety may be present in a *x*:*y* ratio to the matrix-binding domain, where *x* is between 1 and 10, and y is 1. For example, in the embodiment where the matrix-binding domain is a cellulose binding domain, the anti-virus moiety may be present in a 1:1 (equimolar) ratio to the cellulose binding domain. In other embodiments, the ratio may be increased by the specific design to a higher ratio such as 5:1 or 10:1, through multivalent linkage. For example, the use of spider silk with a multivalent linker, such as SpyTag/Spycatcher, would generate a multivalent ratio between the anti-virus moiety and the matrix-binding domain. A higher ratio between an anti-virus moiety and a matrix-binding domain is advantageous because more virus particles and/or fragments thereof may be trapped with a smaller amount of the matrix-binding domain.

The invention also provides a method of immobilising an anti-virus moiety described herein in a matrix. Conditions suitable for the immobilisation are well known in the art and the skilled person would be able to determine such conditions based on teachings in the art.

The matrix-binding domain may be a cellulose-binding domain, a collagen-binding domain, a chitin-binding domain, a mucin-binding domain, or a polymer-binding peptide. Examples of matrix-binding domains and the corresponding matrices are provided in Table 1.

**Table 1. Examples of matrix-binding domains and the corresponding matrices.**

| **Matrix-binding domain** | **Examples of matrix-binding domains** | **Matrix** |
|---|---|---|
| Cellulose-binding domain | Carbohydrate-binding module (CBM) | Cotton, paper, cellulose, starch |
| Collagen-binding domain | | Collagen |
| Chitin-binding domain | | Insect shells |
| Mucin-binding domain | Galectin 3 carbohydrate recognition domain | Mucous membrane |
| Polymer binding peptide | Polystyrene (PS) binding peptide | Polystyrene (PS) |
| | Polypropylene (PP) binding peptide | Polypropylene (PP) (e.g. HEPA filter) |
| | Polyetherimide (PEI) binding peptide | Polyetherimide (PEI) |
| | Hyaluronic acid (HA) binding peptide | Hyaluronic acid (HA) |
| | Hydroxymethacrylate (HEMA) binding peptide | Hydroxymethacryl ate (HEMA) |

As shown in the Examples, the carbohydrate-binding module CBM3 is particularly useful with the invention. Hence, an anti-virus moiety useful with the invention may be coupled (e.g. fused) to CBM3, *e.g.* comprising SEQ ID NO: 6.

The polymer-binding peptides are useful with the invention as they allow attachment of an anti-virus moiety in a non-covalent fashion to various surfaces such as polystyrene (PS), polypropylene (PP) (e.g. HEPA filter), polyetherimide (PEI), hyaluronic acid (HA) or hydroxymethacrylate (HEMA).

### Fusion protein

The invention also provides a fusion protein comprising: (i) an anti-virus moiety, such as an anti-virus moiety described herein, (ii) a matrix-binding domain, and/or (iii) a linker between the anti-virus moiety and the matrix-binding domain. The fusion protein may comprise: (i) an anti-virus moiety, such as an anti-virus moiety described herein, and (ii) a matrix-binding domain. The fusion protein may comprise: (i) an anti-virus moiety described herein, (ii) a matrix-binding domain, and (iii) a linker between the anti-virus moiety and the matrix-binding domain.

The fusion protein may further comprise a solubility tag and/or an affinity tag, as described above.

The anti-virus moiety may specifically bind to an epitope in a coronavirus, such as SARS-CoV-2. The anti-virus moiety may specifically bind to an epitope in a virus (e.g. a coronavirus, such as SARS-CoV-2) with a K_{D} value of between 1 to 10 nM. The anti-virus moiety may comprise an amino acid sequence as described herein, e.g. having ≥70% sequence identity with any one of SEQ ID NOs: 1 to 3.

The matrix-binding domain and the linker between the anti-virus moiety and the matrix-binding domain may be the same protein, e.g. functionalised spider silk. the anti-virus moiety is an antibody, optionally wherein the antibody is a single chain variable fragment (scFv).

"Fusion proteins" as employed herein comprise a protein component fused to one or more protein component.

The fusion protein is a protein expressed by recombinant techniques from a genetic construct, for example expressed in a host from a DNA construct.

The fusion protein may be expressed as a "single protein/unit" from a cell. In the case of a fusion protein comprising a Fab/Fab' fragment there will be two chains but this will be considered a single protein for the purpose of the present specification with one chain.

For example, a fusion protein of the invention comprising: (i) an anti-virus moiety, such as an anti-virus moiety described herein, (ii) a matrix-binding domain, and (iii) a linker between the anti-virus moiety and the matrix-binding domain may be expressed as a "single protein/unit" from a cell.

Alternatively, the fusion protein may be expressed separately from a cell and subsequently fused together, e.g. through spytag technology. For example, a fusion protein of the invention comprising: (i) an anti-virus moiety, such as an anti-virus moiety described herein, (ii) a matrix-binding domain, and (iii) a linker between the anti-virus moiety and the matrix-binding domain may be expressed from separate cells, and subsequently fused together, e.g. through spytag technology.

### Linker

The peptides discussed herein may comprise a linker. The linker allows the peptides to retain biological function by creating a physical distance and/or separation.

The teaching herein of linkers in one context can equally be applied to linkers in different contexts where a linker is employed, such as in a particular anti-virus moiety or a matrix-binding domain described herein. For example, a linker may be used to join the light and heavy chains of the antigen-binding region in a scFv. A linker may be used to join the various components of a fusion protein of the invention, e.g. between the anti-virus moiety and a matrix-binding domain or between the anti-virus moiety and a solubility tag.

Examples of linkers useful with the invention are known in the art, *e.g.* the ones used in the Examples below. Useful linkers include, for example, recombinant spider silk protein, elastin-like polypeptide, troponin C fragment and/or GRG₅RG₄R (GB1-resilin polymer).

In some embodiments, a protein may have dual characteristics, e.g. functionalised spider silk, which may be the linker and the matrix-binding domain.

### Applications and hygiene products

The invention relates to using the anti-virus moiety immobilised in a matrix to prevent virus (*e.g.* SARS-CoV-2) transmission, e.g. by filtering or removing virus particles and/or fragments thereof (*e.g.* SARS-CoV-2) from the environment or a surface.

The immobilised anti-virus moiety may filter or remove ≥10% (i.e. 10% or more), ≥20%, ≥30%, ≥40%, ≥50%, ≥60%, ≥70%, ≥80%, ≥90% or 100% of the virus particles and/or fragments thereof from the environment or a surface.

The immobilised anti-virus moiety may be in contact with the environment or a surface, *e.g*. soil, water or air, for a time and under conditions sufficient for a target virus particle to be retained by the immobilized anti-virus moiety. The retained virus particles may be analysed in situ or removed (e.g. eluted) for further analysis, e.g. biochemically, immunologically or microscopically. Alternatively, the hygiene product containing retained virus particles may simply be discarded.

The matrix of the invention is suitable for use as a hygiene product. For example, the matrix of the invention may be part of the hygiene product. Hence, a hygiene product of the invention may be prepared by incorporating a matrix of the invention.

One of the advantages of the invention is that the matrices described herein can be incorporated in a hygiene product after drying (e.g. air-dried) and storage. Hence, the hygiene product may be a wet or dry product. As demonstrated in the Examples, the anti-virus moiety and fusion proteins of the invention, when immobilized in matrix and then dried, remain active and functional.

In embodiments where the hygiene product is a liquid, the immobilised anti-virus moiety may be at a concentration of between 1 ng/ml and 1 mg/ml (i.e. between 1.63 BVB (billion virus copy binding units) and 1.63 × 10⁶ BVB).

In embodiments where the hygiene product is a solid surface, the immobilised anti-virus moiety may be at a concentration of between 1 ng/cm² to 1 mg/cm² (i.e. between 1.63 BVB (billion virus copy binding units) and 1.63 × 10⁶ BVB). For example, in embodiments where the hygiene product is a bead, the immobilised anti-virus moiety may be at a concentration of between 1 ng/ml and 1 mg/ml (i.e. between 1.63 BVB (billion virus copy binding units) and 1.63 × 10⁶ BVB).

The hygiene product may be a personal protective equipment, such as lab coat, protective mask *(e.g.* face mask, mask, respirator, face shield, surgical mask, medical mask, filter mask, mouth mask, or gas mask), shoe cover, or hair cap.

The hygiene product may be a filter, such as an air filter *(e.g.* HEPA filter) or a water filter.

The hygiene product may comprise a polyethylene membrane.

The hygiene product may be a household product. The household product may be in the form of a sheet, such as tissue, surface wipe, linen, napkin, curtain, or table cloth. The household product may be in the form of a liquid or semi-liquid, such as cleaning solution (*e.g.* aerosol spray), sanitiser gel or cream. The household product may be an infant product, such as diaper, wipe and toy.

The solution may be a cellulose solution.

The invention relates to a method of preventing virus transmission, e.g. by filtering or removing virus particles and/or fragments thereof (*e.g.* SARS-CoV-2) from the environment or a surface, using the immobilised anti-virus moiety of the invention. Similarly, the invention also relates to the use of the immobilised anti-virus moiety of the invention to prevent virus transmission, e.g. to filter or remove virus particles and/or fragments thereof (*e.g.* SARS-CoV-2) from the environment or a surface.

The invention relates to preventing virus transmission in the air.

The immobilised anti-virus moiety of the invention may be used in filters and protective masks to prevent entry of the target virus (*e.g.* SARS-CoV-2) into the respiratory tract. For example, the protective mask may be worn by a subject (e.g. who is not infected by coronavirus *(e.g.* SARS-CoV-2)) to protect himself from coronavirus *(e.g.* SARS-CoV-2) infection.

The immobilised anti-virus moiety of the invention may be used in filters and protective masks to prevent the spread of the target virus (e.g. SARS-CoV-2) into the environment or surfaces from a subject infected by coronavirus (e.g. SARS-CoV-2). For example, the protective mask may be worn by a subject who is infected by coronavirus *(e.g.* SARS-CoV-2) and is wearing the face mask to prevent the spread of coronavirus *(e.g.* SARS-CoV-2) into the environment or surfaces.

Alternatively, the immobilised anti-virus moiety of the invention may be used in aerosol sprays to bind the respiratory droplets that contain the target virus (*e.g.* SARS-CoV-2).

The method or use of the invention may comprise filtering the air through a filter of the invention.

The method or use of the invention may comprise a subject wearing a personal protective equipment of the invention, e.g. a protective mask.

The method or use of the invention may comprise a subject breathing though a protective mask of the invention.

The invention relates to preventing virus transmission in the water. The immobilised anti-virus moiety of the invention may be used in a water filter. The method or use of the invention may comprise filtering water through a filter of the invention.

The invention relates to preventing fomite transmission. The immobilised anti-virus moiety of the invention may be used in a cleaning product, e.g. cleaning solution (*e.g.* aerosol spray), sanitiser gel or cream, to remove the target virus from a surface.

The method or use of the invention may comprise spraying or wiping a hard surface with a cleaning solution (*e.g.* aerosol spray).

The method or use may comprise rubbing sanitiser gel or cream to a body part, e.g. hands.

Typically, the invention relates to methods and uses for a human subject in need thereof. However, non-human animals such as rats, rabbits, sheep, pigs, cows, cats, or dogs is also contemplated.

The subject may be anyone in the population.

The subject may be at risk of exposure to coronavirus infection, such as a healthcare worker or a person who has come into contact with an infected individual. A subject may have visited or be planning to visit a country known or suspected of having a coronavirus outbreak. A subject may also be at greater risk, such as an immunocompromised individual, for example an individual receiving immunosuppressive therapy or an individual suffering from human immunodeficiency syndrome (HIV) or acquired immune deficiency syndrome (AIDS).

The subject may be asymptomatic or pre-symptomatic.

The subject may be early, middle or late phase of the disease.

The subject may be in hospital or in the community at first presentation, and/or later times in hospital.

The subject may be male or female. In certain embodiments, the subject is typically male.

The subject may not have been infected with coronavirus, such as SARS-CoV-2.

The subject may have a predisposition to the more severe symptoms or complications associated with coronavirus infections. The method or use of the invention may comprise a step of identifying whether or not a patient is at risk of developing the more severe symptoms or complications associated with coronavirus.

The subject may or may not have been diagnosed to be infected with coronavirus, such as SARS-CoV-2.

### Composition or combination

A matrix or a hygiene product of the invention may comprise multiple anti-virus moieties, wherein each anti-virus moiety is specific for a different epitope. The different epitopes may be in the same virus or may be in different viruses.

Hence, the invention also provides a composition comprising multiple anti-virus moieties, immobilised in the same or different matrices of the invention. The invention also provides a hygiene product comprising such a composition.

The immobilised anti-virus moiety of the invention may be used in combination with one or more biocides. A biocide may be chlorine dioxide, hydrogen peroxide, peroxy acid, an alcoholic or a phenolic compound, an essential oil and its effective component, bleach, an antibiotic, or an antimicrobial phytochemical. Useful essential oils may include thyme oil, tea tree oil, rosemary oil, eucalyptus oil, or citral oil.

Hence, a matrix or a hygiene product of the invention may comprise the immobilised anti-virus moiety of the invention and a biocide.

A method or use of the invention may comprise applying the immobilised anti-virus moiety of the invention in combination with a biocide to prevent virus transmission (e.g. SARS-CoV-2), e.g. by filtering or removing virus particles and/or fragments thereof from the environment or a surface.

### Other

It is to be understood that different applications of the disclosed immobilised anti-virus moiety, fusion protein and/or matrix of the invention may be tailored to the specific needs in the art. It is also to be understood that the terminology used herein is for the purpose of describing particular embodiments of the invention only, and is not intended to be limiting.

In addition, as used in this specification and the appended claims, the singular forms "a", "an", and "the" include plural references unless the content clearly dictates otherwise. Thus, for example, reference to "an antibody" includes two or more antibodies.

Furthermore, when referring to "≥*x*" herein, this means equal to or greater than x. When referring to "≤*y*" herein, this means equal to or less than *y.*

For the purpose of this invention, in order to determine the percent identity of two sequences (such as two polynucleotide or two polypeptide sequences), the sequences are aligned for optimal comparison purposes (*e.g.* gaps can be introduced in a first sequence for optimal alignment with a second sequence). The nucleotides at each position are then compared. When a position in the first sequence is occupied by the same nucleotide as the corresponding position in the second sequence, then the nucleotides are identical at that position. The percent identity between the two sequences is a function of the number of identical positions shared by the sequences (*i.e.,* % identity = number of identical positions / total number of positions in the reference sequence × 100).

Typically the sequence comparison is carried out over the length of the reference sequence. For example, if the user wished to determine whether a given ("test") sequence is 95% identical to SEQ ID NO: 3, SEQ ID NO: 3 would be the reference sequence. To assess whether a sequence is at least 95% identical to SEQ ID NO: 3 (an example of a reference sequence), the skilled person would carry out an alignment over the length of SEQ ID NO: 3, and identify how many positions in the test sequence were identical to those of SEQ ID NO: 3. If at least 95% of the positions are identical, the test sequence is at least 95% identical to SEQ ID NO: 3. If the sequence is shorter than SEQ ID NO: 3, the gaps or missing positions should be considered to be non-identical positions.

The skilled person is aware of different computer programs that are available to determine the homology or identity between two sequences. For instance, a comparison of sequences and determination of percent identity between two sequences can be accomplished using a mathematical algorithm. In an embodiment, the percent identity between two amino acid or nucleic acid sequences is determined using the Needleman and Wunsch (1970) algorithm which has been incorporated into the GAP program in the Accelrys GCG software package (available at http://www.accelrys.com/products/gcg/), using either a Blosum 62 matrix or a PAM250 matrix, and a gap weight of 16, 14, 12, 10, 8, 6, or 4 and a length weight of 1, 2, 3, 4, 5, or 6.

All publications, patents and patent applications cited herein, whether supra or infra, are hereby incorporated by reference in their entirety.

The following examples illustrate the invention.

### Example 1

This experiment aims to express and purify anti-virus moieties, each coupled to a cellulose-binding domain, also referred to herein as Karunyam proteins. The binding properties of each of the Karunyam proteins are also investigated.

### Materials and methods

### DNA constructs

Three Karunyam proteins, each containing a scFv coupled to a matrix-binding domain (cellulose binding motif 3, CBM3), were prepared. scFv sequences were synthesised from a commercial source (Genscript) with the format VH-linker-VL, based on available published sequences of monoclonal antibodies P2B2F6, P2C1F11 and P2C1A3 as disclosed in References 4 and 5. A parent vector was created using a cold-induction vector (pCOLD-I), with an N-terminal histidine tag and glutathione-S-transferase solubility enhancement tag. At the C-terminal, a cellulose binding motif (CBM3) was introduced. scFv constructs were cloned in frame into the parent vector to create the bacterial expression vectors. The resulting Karunyam proteins have the sequences set out in Table 2.

**Table 2: Features of the Karunyam proteins.**

| **Karunyam protein** | **Anti-virus moiety** | **N-terminal tag** | **Matrix binding protein at C terminus** |
|---|---|---|---|
| Karunyam 9 (SEQ ID NO: 7) | scFv 9 (SEQ ID NO: 1) | His-tag (SEQ ID NO: 4) and GST-tag (SEQ ID NO: 5) | CBM3 (SEQ ID NO: 6) |
| Karunyam 10 (SEQ ID NO: 8) | scFv 10 (SEQ ID NO: 2) | | |
| Karunyam 11 (SEQ ID NO: 9) | scFv 11 (SEQ ID NO: 3) | | |

### Protein expression and affinity purification

DNA constructs were expressed in the BL21 (DE3) strain of *E. coli.* An overnight culture of bacterial culture was plated to create a confluent plate. Half of the cells from a confluent plate were scraped off and resuspended in 100mL of 2X YT broth. After growth at 37°C for 3 hours, the cells were cooled on ice for 30 minutes, IPTG was added to 0.5mM, and the cell were allowed to incubate with shaking at 15°C for 21 hours. The cells were centrifuged into a pellet, then resuspended in lysis buffer (8mL PBS, 2mL PopCulture reagent, 2µL Lyonase). The resuspended cells were disrupted using three freeze-thaw cycles using liquid nitrogen. A combination of GST magnetic beads and Nickel-NTA magnetic beads (250µL each) were added to the culture, with stirring at room temperature for 30 minutes. The magnetic beads were denatured overnight at 4°C in a mild denaturing solution (2M urea, ImM DTT, 0.05% Tween 20). The next day, the refolding process was carried out with two successive 20 minute incubations with 1M and 0.5M urea solutions containing ImM DTT and 0.05% Tween 20 as well. The protein was eluted first with an elution buffer containing 15mM Glutathione, then with a second elution buffer containing 500mM imidazole.

To improve the expression and solubility of the scFvs, the following reagents and conditions were tested: choice of cell strain, choice of N-terminal solubility tags, optimization of protein induction time, optimization of IPTG concentration during induction, and use of appropriate solubilization and denaturation/refolding buffers. The results are summarised below:
1. Use of different *E*. *Coli* strains to optimise expression: BL21 (DE3)LysS, BL21(DE3), DRX, and NovaBlue were used. The best expression strain was found to be BL21(DE3) which was used for further experiments.
2. Solubility tags were attached to the N-terminus of the fusion proteins, including GB1, GST, SUMO, MBP and NusA. The GST fusion proteins showed greatest solubility and ease of purification and were used for further experiments.
3. Induction time at 15 degrees Celsius was optimised from 6 hours to 21 hours. The final experimental conditions involved 3 hours of cell growth at 37 degrees, followed by induction at 15 degrees for 21 hours.
4. IPTG concentration was optimized to 0.5 mM during the protein induction phase at 15 degrees Celsius.
5. Solubilization and denaturation/renaturation were carried out using a variety of buffers. The best yield was obtained using the conditions described above.

### Far-western blot analysis

Affinity purified Karunyam proteins were immobilised on nitrocellulose (NC) membranes using a slot-blot apparatus. Serial dilutions of a control monoclonal antibody to the RBD of the spike protein of SARS-CoV-2 was added to 4 wells as a positive control. The NC blot was dried for one hour, blocked with 5% milk in TBST, and treated overnight at 4°C with a solution of RBD-spike protein conjugated to HRP (Genscript; amino acids 319-Ser591). The next day, the blot was washed three times with TBST, then developed using ECL imaging.

### Enzyme-linked immunosorbent assay (ELISA)

Affinity-purified Karunyam proteins were coated overnight on glutathione-lined 96-well ELISA plates (200ng/100µL) at 4°C. The next day, plates were washed twice with Tris-buffered Saline pH 8.0 containing 0.1% Tween-20 (TBST), then allowed to incubate with serial dilutions of biotinylated RBD-spike protein/Streptavidin-HRP mixture for 1 hour at 37°C. The biotinylated RBD spike was from BIOSSUSA, amino acids Arg319-Ans532. Plates were washed twice with TBST. 3,3',5,5'-tetramethylbenzidine (TMB, Fisher) solution was added for 15 minutes at room temperature, and then Stop solution (0.16M sulfuric acid, Fisher) was added to quench the enzyme reaction. Plates were read at 450nM using a Cytation 5 image reader.

### Results

Vectors containing sequences encoding the Karunyam proteins were prepared in bacterial cultures and vector expression was induced. Bacterial cultures were then lysed by freeze-thaw cycles. Karunyam proteins were unfolded and refolded using denaturants, and then affinity purified using nickel magnetic beads and glutathione magnetic beads. As shown in **Figure 1****,** four preparations of Karunyam 9 were affinity purified and analysed by 4-12% SDS-PAGE. Lane 1 shows the molecular weight markers in kDa. Lane 2 shows a sample of pre-induction cell lysate. Lanes 3-6 show cell lysates after vector expression was induced by cold induction. Lanes 7-10 show the affinity purified protein. The black arrows indicate the band that corresponds to the size of the Karunyam 9 protein. The gel in **Figure 1** therefore demonstrates that Karunyam 9 can be expressed from bacteria and that subsequent affinity purification successfully enriches Karunyam 9 from cell lysate.

To test whether the Karunyam proteins could bind to the RBD-spike protein, the affinity purified Karunyam proteins were immobilised on a nitrocellulose membrane and RBD-spike protein conjugated to HRP was added. Detection of signal during ECL imaging is indicative of specific binding of the RBD-spike protein to the Karunyam protein. Results of the far-western blot assay are shown in **Figure 2A****.** Three distinct bands were detected on the blot, corresponding to Karunyam 9, Karunyam 10 and Karunyam 11. Accordingly, Karunyam 9, Karunyam 10 and Karunyam 11, when immobilised on a membrane, were all capable of binding to the RBD-spike protein.

To quantify the binding affinity of the Karunyam proteins to the RBD-spike protein, an ELISA was performed according to the method described above. **Figure 2B** shows a dose response curve for Karunyam 9 with values of optical density at 450nM against increasing concentration of RBD-spike protein. The K_{D} for each Karunyam protein was estimated from dose response curves, and is shown in **Table 3** below. For comparison, **Table 4** shows the K_{D} for the known antibodies from which the scFvs were derived, and the K_{D} for the human Angiotensin-converting enzyme 2 (ACE2) receptor which naturally binds to the RBD. As shown in **Table 3,** each of Karunyam 9, Karunyam 10 and Karunyam 11 binds to the RBD-spike protein with high affinity. The binding affinity of these proteins for the RBD-spike protein is similar to that of the human ACE2 receptor for the RBD-spike protein of SARS-CoV-2, and to that of the known antibodies on which they are based.

**Table 3: Binding affinity of Karunyam proteins at 37°C**

| **Karunyam** | **Anti-virus moiety** | **K_{D} (nM)** |
|---|---|---|
| Karunyam 9 | scFv 9 | 4.05 + 0.26 |
| Karunyam 10 | scFv 10 | 6.35 + 0.65 |
| Karunyam 11 | scFv 11 | 7.88 + 2.06 |

**Table 4: Binding affinity of known antibodies of the prior art at 37°C**

| **Anti-virus moiety** | **Parent antibody** | **K_{D} (nM)** | **Reference** |
|---|---|---|---|
| scFv 9 | P2B-2F6 | 5.14 | 5 |
| scFv 10 | P2C-1F11 | 2.12 | 5 |
| scFv 11 | P2C-1A3 | 2.47 | 5 |
| ACE2 receptor | N/A | 4.7 to SARS-CoV2-RBD 31 to SARS-CoV-RBD | 4 |

### Example 2

This experiment aims to elucidate whether the Karunyam proteins, when immobilised on cellulose beads, can bind to viral pseudoparticles in solution. Viral pseudoparticles consist of the RBD-spike protein conjugated to FITC nanoparticles, which have a diameter of approximately 100nm. Coronavirus particles have a diameter of approximately 60-140nm, so these viral pseudoparticles act as a model for the coronavirus particles.

### Materials and methods

### Cellulose bead binding assay

Cellulose beads of 1mm average diameter were coated overnight with purified Karunyam protein at 4°C, using 200ng of Karunyam protein in a total volume of 1mL PBS in each well of a 12 well culture plate. One in two serial dilutions were prepared of RBD-spike protein conjugated to FITC nanoparticles (viral pseudoparticles). 60µL of each dilution was estimated for RBD-spike protein using immunochromatography (IC) strips. The reactivity was estimated after 15 minutes. Beads were washed twice with TBST and then treated for one hour at 37°C with serial dilutions of RBD-spike protein conjugated with FITC nanoparticles. The unbound RBD-spike protein content was estimated using IC strips.

For RBD-spike protein content, RBD spike protein dilutions in 100uL TBST were prepared ranging from 20nM to 0.15 nM. 60uL of each dilution was spotted on the IC strip and allowed to develop over 15 minutes. The presence of the positive band indicates the presence of RBD-spike protein. The threshold sensitivity of the IC strip was determined under these conditions as 0.63 nM.

The cellulose beads with immobilised Karunyam protein were added to the serial dilutions of RBD-spike protein. The RBD-spike protein dilutions were then re-tested using the IC strips to estimate the amount of unbound RBD-spike protein after treatment with the beads. Table 5 shows the serial dilutions of RBD-spike protein and the qualitative IC strip results before and after treatment with immobilised Karunyam proteins. The difference in IC testing between pre- and post-Karunyam treatment RBD-spike protein provided the approximate amounts of RBD-spike protein bound to immobilised Karunyam proteins.

### Results

To test whether the Karunyam proteins when bound to a cellulose matrix can bind to virus pseudoparticles, cellulose bead binding assays were performed. Karunyam proteins immobilised on cellulose beads were treated with serial dilutions of RBD-spike protein conjugated with FITC nanoparticles. The FITC nanoparticles have a diameter of 100nm so are representative of coronavirus particles, which are 60-140nm in diameter. As shown in **Figures 3A and B****,** fluorescence of the cellulose beads was detected after 5 minutes, demonstrating that the Karunyam proteins bound to the cellulose beads can bind the RBD-spike protein conjugated with FITC nanoparticles. When left in solution for one week, the Karunyam proteins bound to the cellulose beads continued to bind the RBD-spike protein, as demonstrated in **Figures 3C and D****.**

To estimate the amount of RBD-spike protein bound to the Karunyam proteins immobilised on the cellulose beads, IC strips were used to firstly estimate the amount of total RBD-spike protein in the serial dilutions, and then to estimate the amount of unbound RBD-spike protein after addition to the beads. The difference between total and unbound RBD-spike protein provided the approximate amounts of RBD-spike protein bound to immobilised Karunyam proteins, as shown in **Table 5.**

**Table 5: Estimated amount of RBD-spike protein bound to Karunyam proteins immobilised on cellulose beads after 15 minutes.**

| | **Dilution of RBD-spike protein** | | | | | | |
|---|---|---|---|---|---|---|---|
| | 10nM | 5nM | 2.5nM | 1.25nM | 0.63 nM | 0.31 nM | 0.15nM |
| **TOTAL** | + | + | + | + | + (0.63) | - | - |
| **Karunyam 9-unbound** | + | + (0.63) | - | - | - | - | - |
| **Karunyam 10-unbound** | + | + (0.63) | - | - | - | - | - |
| **Karunyam 11-unbound** | + | + (0.63) | - | - | - | - | - |

The + sign indicates the presence of a positive signal on the IC strip. The - sign indicates absence of a signal on the IC strip.

### Example 3

This experiment investigates the extent to which the Karunyam proteins, when immobilised on cellulose beads, can retain virus pseudoparticles.

Karunyam proteins bound to cellulose beads were subject to the virus pseudoparticle trap assay. In detail, cellulose beads of 1mm average diameter were coated overnight with Karunyam protein at 4°C, using 200ng of Karunyam protein in a total volume of 0.5mL PBS in a spin-column. RBD-spike protein conjugated to FITC nanoparticles (virus pseudoparticles) at a concentration of 10nM were incubated in each spin column in a volume of 200µL for 30 minutes at 37°C. The spin columns were centrifuged at 14,000g for 5 minutes. The unbound RBD-spike protein fraction was determined using a competitive ELISA. The difference between total and unbound RBD-spike protein as provided by IC analysis provides a value of the RBD-spike protein bound to cellulose beads.

For competitive ELISA, affinity-purified SCFVs were coated overnight on Glutathione plates (200 ng/100ul) at 4oC. The next day, plates were washed twice with Tris-buffered Saline pH 8.0 containing 0.1% Tween-20 (TBST), then allowed to incubate with serial dilutions of biotinylated RBD-Spike protein/Streptavidin-HRP mixture for 1 hour at 37oC, in the presence/absence of competitor (Total Spike-RBD/FITC added to spin-column (Total), or Unbound Spike-RBD/FITC eluate from the spin-column (Unbound)).

Plates were washed twice with TBST. 3,3',5,5' - tetramethylbenzidine (TMB, Fisher) solution was added for 15 minutes at room temperature, and then Stop solution (0.16M sulfuric acid, Fisher)) was added to quench the enzyme reaction. Plates were read at 450nM using a Cytation 5 image reader. The amount of unbound Spike-RBD/FITC was determined as the difference between the Total -Unbound.

The results of the pseudoparticle trap assay are shown in **Figure 4****.** Karunyam 9, Karunyam 10 and Karunyam 11 all retained a large proportion of the total virus pseudoparticles after centrifugation. On average, the percentage of total virus pseudoparticles that are retained by the Karunyam proteins is 93%.

These results show that Karunyam 9, Karunyam 10 and Karunyam 11 immobilised on cellulose beads are able to retain virus pseudoparticles after centrifugation. Accordingly, these proteins can act as "traps" for virus particles and/or fragments thereof.

### Example 4

Karunyam 9, Karunyam 10 or Karunyam 11 is immobilised in a face mask at a concentration of about 1 ng/cm². The face mask is used by a COVID19 patient. The face mask is capable of sequestering SARS-CoV-2 exhaled from the patient for approximately 900 hours.

The face mask is worn by a COVID19 patient in hospitals and enclosed places, such as aircraft, to prevent SARS-CoV-2 transmission and protect others from SARS-CoV-2 infections.

### References

1 Holliger and Hudson, (2005) Nature Biotech. 23(9):1126-1136.
2 Adair and Lawson, (2005) Drug Design Reviews - Online 2(3), 209-217.
3 Verma et al., (1998) Journal of Immunological Methods, 216, 165-181.
4 Lan et al., (2020) Nature, 581:215-220
5 Ju et al., (2020) Nature, 584:115-119

### Sequence listing

**SEQ ID NO: 1 ―** scFv 9 
DPIVMIDNDKPITGGGGSGSQVQLQESGPGLVKPSETLSLTCTVSGYSISSGYYWG
WIRQPPGKGLEWIGSIYHSGSTYYNPSLKTRVTISVDTSKNQFSLKLSSVTAADTAV
YYCARAVVGIVVVPAAGRRAFDIWGQGTMVTVSSGGGGSGGGGSGGGGSGGGG
SGGGGSQSALTQPPSASGSPGQSVTISCTGTSSDVGGYNYVSWYQQHPGKAPKLM
IYEVSKRPSGVPDRFSGSKSGNTASLTVSGLQAEDEADYYCSSYAGSNNLVFGGGT
KV
**SEQ ID NO: 2** - scFv 10 
AHIVMVDAYKPTKGGGGSGEVQLVESGGGLVQPGGSLRLSCAASGITVSSNYM
NWVRQAPGKGLEWVSLIYSGGSTYYADSVKGRFTISRDNSKNTLYLQMNSLRAE
DTAVYHCARDLVVYGMDVWGQGTTVTVSSGGGGSGGGGSGGGGSGGGGSGGG
GSEIVLTQSPGTLSLSPGERATLSCRASQSVSSSYLAWYQQKPGQAPRLLIYGASSR
ATGIPDRFSGSGSGTDFTLTISRLEPEDFAVYYCQQYGSSPTFGQGTKLEIK
**SEQ ID NO: 3** ― scFv 11 
KLGDIEFIKVNKGGGGSGSQVQLVESGGGLVKPGGSLRLSCAASGFTFSDYYMSW
IRQAPGKGLEWVSYISSSGSTIYYADSVKGRFTISRDNAKNSLYLQMNSLRAEDTA
VYYCARDFSHQQLVPSWGQGTLVTVSSGGGGSGGGGSGGGGSGGGGSGGGGSDI
QLTQSPSFLSASVGDRVTITCRASQGISSYLAWYQQKPGKAPKLLIYAASTLQSGV
PSRFSGSGSGTEFTLTISSLQPEDFATYYCQQLNSYPLTFGGGTKVEIK
**SEQ ID NO: 4** ― Histidine affinity tag
   HHHHHH
**SEQ ID NO: 5 ―** Glutathione-S-transferase (GST) solubility tag. 
SPILGYWKIKGLVQPTRLLLEYLEEKYEEHLYERDEGDKWRNKKFELGLEFPNLPY
YIDGDVKLTQSMAIIRYIADKHNMLGGCPKERAEISMLEGAVLDIRYGVSRIAYSK
DFETLKVDFLSKLPEMLKMFEDRLCHKTYLNGDHVTHPDFMLYDALDVVLYMDP
MCLDAFPKLVCFKKRIEAIPQIDKYLKSSKYIAWPLQGWQATFGGGDHPPKGIEEN
LYFQS
**SEQ ID NO: 6** ― Cellulose binding domain 3(CBM3) 
VSGNLKVEFYNSNPSDTTNSINPQFKVTNTGSSAIDLSKLTLRYYYTVDGQKDQTF
WCDHAAIIGSNGSYNGITSNVKGTFVKMSSSTNNADTYLEISFTGGTLEPGAHVQI
QGRFAKNDWSNYTQSNDYSFKSASQFVEWDQVTAYLNGVLVWGKESR
**SEQ ID NO: 7 ―** Karunyam 9 
MNHKVHHHHHHIEGRHMSPILGYWKIKGLVQPTRLLLEYLEEKYEEHLYERDEG
DKWRNKKFELGLEFPNLPYYIDGDVKLTQSMAIIRYIADKHNMLGGCPKERAEIS
MLEGAVLDIRYGVSRIAYSKDFETLKVDFLSKLPEMLKMFEDRLCHKTYLNGDHV
THPDFMLYDALDVVLYMDPMCLDAFPKLVCFKKRIEAIPQIDKYLKSSKYIAWPL
QGWQATFGGGDHPPKGIEENLYFQSHMELGTLEGEFDPIVMIDNDKPITGGGGSGS
QVQLQESGPGLVKPSETLSLTCTVSGYSISSGYYWGWIRQPPGKGLEWIGSIYHSG
STYYNPSLKTRVTISVDTSKNQFSLKLSSVTAADTAVYYCARAVVGIVVVPAAGR
RAFDIWGQGTMVTVSSGGGGSGGGGSGGGGSGGGGSGGGGSQSALTQPPSASGS
PGQSVTISCTGTSSDVGGYNYVSWYQQHPGKAPKLMIYEVSKRPSGVPDRFSGSK
SGNTASLTVSGLQAEDEADYYCSSYAGSNNLVFGGGTKVDSVSGNLKVEFYNSNP
SDTTNSINPQFKVTNTGSSAIDLSKLTLRYYYTVDGQKDQTFWCDHAAIIGSNGSY
NGITSNVKGTFVKMSSSTNNADTYLEISFTGGTLEPGAHVQIQGRFAKNDWSNYT
QSNDYSFKSASQFVEWDQVTAYLNGVLVWGKESR
**SEQ ID NO: 8** ― Karunyam 10 
MNHKVHHHHHHIEGRHMSPILGYWKIKGLVQPTRLLLEYLEEKYEEHLYERDEG
DKWRNKKFELGLEFPNLPYYIDGDVKLTQSMAIIRYIADKHNMLGGCPKERAEIS
MLEGAVLDIRYGVSRIAYSKDFETLKVDFLSKLPEMLKMFEDRLCHKTYLNGDHV
THPDFMLYDALDVVLYMDPMCLDAFPKLVCFKKRIEAIPQIDKYLKSSKYIAWPL
QGWQATFGGGDHPPKGIEENLYFQSHMELGTLEGEFAHIVMVDAYKPTKGGGGS
GSEVQLVESGGGLVQPGGSLRLSCAASGITVSSNYMNWVRQAPGKGLEWVSLIYS
GGSTYYADSVKGRFTISRDNSKNTLYLQMNSLRAEDTAVYHCARDLVVYGMDV
WGQGTTVTVSSGGGGSGGGGSGGGGSGGGGSGGGGSEIVLTQSPGTLSLSPGERA
TLSCRASQSVSSSYLAWYQQKPGQAPRLLIYGASSRATGIPDRFSGSGSGTDFTLTI
SRLEPEDFAVYYCQQYGSSPTFGQGTKLEIKDSVSGNLKVEFYNSNPSDTTNSINPQ
FKVTNTGSSAIDLSKLTLRYYYTVDGQKDQTFWCDHAAIIGSNGSYNGITSNVKGT
FVKMSSSTNNADTYLEISFTGGTLEPGAHVQIQGRFAKNDWSNYTQSNDYSFKSA
SQFVEWDQVTAYLNGVLVWGKESR
**SEQ ID NO: 9** ― Karunyam 11 
MNHKVHHHHHHIEGRHMSPILGYWKIKGLVQPTRLLLEYLEEKYEEHLYERDEG
DKWRNKKFELGLEFPNLPYYIDGDVKLTQSMAIIRYIADKHNMLGGCPKERAEIS
 
MLEGAVLDIRYGVSRIAYSKDFETLKVDFLSKLPEMLKMFEDRLCHKTYLNGDHV
THPDFMLYDALDVVLYMDPMCLDAFPKLVCFKKRIEAIPQIDKYLKSSKYIAWPL
QGWQATFGGGDHPPKGIEENLYFQSHMELGTLEGEFKLGDIEFIKVNKGGGGSGS
QVQLVESGGGLVKPGGSLRLSCAASGFTFSDYYMSWIRQAPGKGLEWVSYISSSG
STIYYADSVKGRFTISRDNAKNSLYLQMNSLRAEDTAVYYCARDFSHQQLVPSWG
QGTLVTVSSGGGGSGGGGSGGGGSGGGGSGGGGSDIQLTQSPSFLSASVGDRVTIT
CRASQGISSYLAWYQQKPGKAPKLLIYAASTLQSGVPSRFSGSGSGTEFTLTISSLQ
PEDFATYYCQQLNSYPLTFGGGTKVEIKDSVSGNLKVEFYNSNPSDTTNSINPQFK
VTNTGSSAIDLSKLTLRYYYTVDGQKDQTFWCDHAAIIGSNGSYNGITSNVKGTFV
KMSSSTNNADTYLEISFTGGTLEPGAHVQIQGRFAKNDWSNYTQSNDYSFKSASQ
FVEWDQVTAYLNGVLVWGKESR
SEQ ID NO: 10 - FR1 of the heavy chain variable domain of scFv 9 QVQLQESGPGLVKPSETLSLTCTVS
SEQ ID NO: 11 ― CDR1 of the heavy chain variable domain of scFv 9 GYSISSGYY
SEQ ID NO: 12 - FR2 of the heavy chain variable domain of scFv 9 WGWIRQPPGKGLEWIGS
SEQ ID NO: 13 - CDR2 of the heavy chain variable domain of scFv 9 IYHSGST
SEQ ID NO: 14 - FR3 of the heavy chain variable domain of scFv 9 YYNPSLKTRVTISVDTSKNQFSLKLSSVTAADTAVYYC
SEQ ID NO: 15 - CDR3 of the heavy chain variable domain of scFv 9 ARAVVGIVVVPAAGRRAFDIW
SEQ ID NO: 16 - FR1 of the light chain variable domain of scFv 9 QSALTQPPSASGSPGQSVTISCTGT
SEQ ID NO: 17 - CDR1 of the light chain variable domain of scFv 9 SSDVGGYNY
SEQ ID NO: 18 - FR2 of the light chain variable domain of scFv 9 VSWYQQHPGKAPKLMIY
SEQ ID NO: 19 - CDR2 of the light chain variable domain of scFv 9 EVS
SEQ ID NO: 20 - FR3 of the light chain variable domain of scFv 9 KRPSGVPDRFSGSKSGNTASLTVSGLQAEDEADYYC
SEQ ID NO: 21 - CDR3 of the light chain variable domain of scFv 9 SSYAGSNNLVFGGGTKVD
SEQ ID NO: 22 - FR1 of the heavy chain variable domain of scFv 10 EVQLVESGGGLVQPGGSLRLSCAAS
SEQ ID NO: 23 - CDR1 of the heavy chain variable domain of scFv 10 GITVSSNY
SEQ ID NO: 24 - FR2 of the heavy chain variable domain of scFv 10 MNWVRQAPGKGLEWVSL
SEQ ID NO: 25 - CDR2 of the heavy chain variable domain of scFv 10 IYSGGSTY
SEQ ID NO: 26 - FR3 of the heavy chain variable domain of scFv 10 YADSVKGRFTISRDNSKNTLYLQMNSLRAEDTAVYHC
SEQ ID NO: 27 - CDR3 of the heavy chain variable domain of scFv 10 ARDLVVYGMDVWGQGTTVTV
SEQ ID NO: 28 - FR1 of the light chain variable domain of scFv 10 EIVLTQSPGTLSLSPGERATLSC
SEQ ID NO: 29 - CDR1 of the light chain variable domain of scFv 10 RASQSVSSSY
SEQ ID NO: 30 - FR2 of the light chain variable domain of scFv 10 LAWYQQKPGQAPRLLIY
SEQ ID NO: 31 - CDR2 of the light chain variable domain of scFv 10 GAS
SEQ ID NO: 32 - FR3 of the light chain variable domain of scFv 10 SRATGIPDRFSGSGSGTDFTLTISRLEPEDFAVYYCQQYGSSPTFGQGTKLEIKVD
SEQ ID NO: 33 - CDR3 of the light chain variable domain of scFv 10 QQYGSSPTFGQGTKLEIKVD
SEQ ID NO: 34 - FR1 of the heavy chain variable domain of scFv 11 QVQLVESGGGLVKPGGSLRLSCAAS
SEQ ID NO: 35 - CDR1 of the heavy chain variable domain of scFv 11 GFTFSDYY
SEQ ID NO: 36 - FR2 of the heavy chain variable domain of scFv 11 MSWIRQAPGKGLEWVSY
SEQ ID NO: 37 - CDR2 of the heavy chain variable domain of scFv 11 ISSSGSTI
SEQ ID NO: 38 - FR3 of the heavy chain variable domain of scFv 11 YYADSVKGRFTISRDNAKNSLYLQMNSLRAEDTAVYYC
SEQ ID NO: 39 - CDR3 of the heavy chain variable domain of scFv 11 ARDFSHQQLVPSWGQGTLVTV
SEQ ID NO: 40 - FR1 of the light chain variable domain of scFv 11 DIQLTQSPSFLSASVGDRVTITCRAS
SEQ ID NO: 41 - CDR1 of the light chain variable domain of scFv 11 QGISSY
SEQ ID NO: 42 - FR2 of the light chain variable domain of scFv 11 LAWYQQKPGKAPKLLIY
SEQ ID NO: 43 - CDR2 of the light chain variable domain of scFv 11 AAS
SEQ ID NO: 44 - FR3 of the light chain variable domain of scFv 11 TLQSGVPSRFSGSGSGTEFTLTISSLQPEDFATYYC
SEQ ID NO: 45 - CDR3 of the light chain variable domain of scFv 11 QQLNSYPLTFGGGTKVEIKVD

## Claims

1. An anti-virus moiety immobilised in a matrix, wherein the matrix is suitable for use as a hygiene product.

2. The anti-virus moiety of claim 1, wherein the anti-virus moiety is an antibody, optionally wherein the antibody is a single chain variable fragment (scFv).

3. The anti-virus moiety of claim 1 or claim 2, wherein the anti-virus moiety specifically binds to an epitope in a coronavirus, such as SARS-CoV-2, with a K_{D} value of between 1 to 10 nM.

4. The anti-virus moiety of any one of the preceding claims, wherein the anti-virus moiety comprises an amino acid sequence having ≥70% sequence identity with any one of SEQ ID NOs: 1 to 3.

5. The anti-virus moiety of any one of the preceding claims, wherein the matrix is cellulose, starch, collagen, insect shells, mucous membrane, or an inorganic matrix.

6. The anti-virus moiety of any one of the preceding claims, wherein the anti-virus moiety is coupled to a matrix-binding domain.

7. The anti-virus moiety claim 7, wherein the matrix is cellulose and the matrix-binding domain comprises SEQ ID NO: 6.

8. The anti-virus moiety of any one of the preceding claims, wherein the matrix is part of the hygiene product.

9. A fusion protein comprising: (i) the anti-virus moiety as defined in any one of claims 2 to 4, and (ii) a matrix-binding domain; optionally wherein the fusion protein further comprises a linker between the anti-virus moiety and the matrix-binding domain.

10. A matrix comprising the anti-virus moiety of any one of claims 1 to 8 or the fusion protein of claim 9 immobilised therein, optionally wherein the matrix is cellulose, starch, collagen, insect shells, mucous membranes, or an inorganic matrix.

11. A hygiene product comprising the anti-virus moiety of any one of claims 1 to 8, the fusion protein of claim 9, or the matrix of claim 10.

12. The anti-virus moiety, the fusion protein, the matrix or the hygiene product of any one of the preceding claims, wherein the hygiene product is:
(a) a personal protective equipment, such as lab coat, protective mask (e.g. face mask, mask, respirator, face shield, surgical mask, medical mask, filter mask, mouth mask, or gas mask), shoe cover, or hair cap;
(b) a filter, such as an air filter *(e.g.* HEPA filter) or a water filter;
(c) a polyethylene membrane;
(d) a household product in the form of a sheet, such as such as tissue, surface wipe, linen, napkin, curtain, or table cloth;
(e) a household product in the form of a liquid or semi-liquid, such as cleaning solution (e.g. aerosol spray), sanitiser gel or cream; or
(f) a cellulose solution.

13. A method of filtering or removing virus particles and/or fragments thereof from the environment or a surface, comprising applying the anti-virus moiety, the fusion protein, the matrix, or the hygiene product of any one of the preceding claims to filter or remove virus particles and/or fragments thereof from the environment or a surface, optionally wherein the virus is SARS-CoV-2.

14. Use of the anti-virus moiety, the fusion protein, the matrix or the hygiene product of any one of the preceding claims to filter or remove virus particles and/or fragments thereof from the environment or a surface, optionally wherein the virus is SARS-CoV-2.

15. A method of preventing virus transmission comprising filtering or removing virus particles and/or fragments thereof from the environment or a surface according to the method or use of claim 13 or 14, optionally wherein the virus is SARS-CoV-2.
